# EUROPEAN PATENT APPLICATION

(11) **EP 3 831 212 A1**
(43) Date of publication of application: **09.06.2021**
(21) Application number: 19927575.1
(22) Date of filing: 24.10.2019
(51) Int. Cl.: A23J 3/14, A23J 1/12, A23K 10/38, A23K 40/25, B02C 7/175, C12C 7/28

(54) **PROTEIN SUSPENSION PRODUCED FROM BREWER'S SPENT GRAIN, METHOD AND APPARATUS FOR PRODUCING SAME**

(30) Priority: 21.10.2019 RU 2019133308
(71) Applicant: BioBo GmbH, 95460 Bad Berneck (DE)
(72) Inventor: GORDILOV, Oleg Grigorievich, Rostov-na-Donu (RU)
(74) Representative: Spengler, Robert
(86) International application number: PCT/RU2019/000766
(87) International publication number: WO 2021/080450

(57) **Abstract**

The present technology generally relates to food-processing industry, namely to a method and a machine for processing brewer's grains.

## Description

### FIELD OF TECHNOLOGY

The present technology generally relates to a protein suspension obtained from spent barley grains, as well as to methods and apparatus for processing brewer's grains in order to obtain protein product in the form of suspension.

### BACKGROUND INFORMATION

After producing beer, brewing companies have a lot of wastes in the form of brewer's grains consisting of remains of barley glume and grain particles rich in protein and fat. This brewer's (malted barley) grains raise the greatest interest among all raw material resources from the brewing industry because they are produced in huge volumes and contain valuable nutritional ingredients.

Brewer's grains are obtained at the stage of filtering saccharified brewer's mash. Percentage of brewer's grains in the brewing industry wastes amounts to at least 98%. Brewer's grains consist of liquid and solid phases. The solid phase that accounts for approximately 45% in brewer's grains is in the form of glume and grain kernel particles. Spent barley grains are rich in fats, fibres and amino acids: histidine, lysine, leucine, isoleucine, methionine, valine, glycine, threonine, serine, alanine, arginine, phenylalanine, tyrosine etc. Every year Russian brewing corporations dispose more than 3.5 mln tons of brewer's grains - its protein content amounts to 25-28% which is almost 3 times higher than the protein amount in barley. In terms of calories wet grains contain 115 cal/g and dry grains contain up to 440 cal/g (with the humidity level of 7-10%). Ingredients in brewer's grains depend on the type of barley, technologies used for manufacturing brewer's malt, malt mix recipe for manufacturing beer, recipes for manufacturing beer etc. However, the obtained quantitative composition of proteins, fats, carbohydrates and fibres in brewer's grains varies slightly from 1 to 5%.

At present, spent barley grains in the original form are not extensively used because transportation and storage processes are very complicated - fermentation process starts in 6-8 hours at the temperature of 15-30°C and the grains become unsuitable for processing and further use.

Technological solutions offer various methods of processing brewer's grains for use as a feed supplement based on their preliminary frying with further granulation or grinding (for example, EP 0694609A2; WO 2010053493A1; WO 2010117288A1; WO 9822751A1, all incorporated herein by reference). However, in the process of drying part of protein ingredients is transformed into non-digestible mass which causes decrease in nutritional value of dry brewer's grains in comparison to wet brewer's grains. The protein content in dried brewer's grains reaches only 27-28%. This product also contains significant amount (up to 80%) of non-digestible malted barley husk. Drying brewer's grains also requires great power consumption so it is not always economically reasonable to make animal feed from such grains.

Known methods also include deeper processing of waste from the brewing industry. In particular, there is a known method of processing liquid brewer's grains with the humidity level of 90-92% that envisages processing of raw materials using two-step compression method : to the humidity level of 70-75% during the first step and to the humidity level of 40-45% during the second step, and then two-stage drying process: to the humidity level of 20-25% during the first stage and to the humidity level of 10% during the second stage with obtaining dry feed supplement (RU 2215426, incorporated herein by reference). This method has a flaw: centrate containing a significant amount of nutrients is removed in the course of compression. Besides, the final product is also high in the amount of barley husk in the product.

Another known method suggests deriving a protein product from brewer's grains with the protein content from 60% to 90% (WO 2018136234A1, incorporated herein by reference). This method implies thermochemical processing of brewer's grains when the following is done: a mixture of spent grains and water is added in a hydrolysis tank with constant stirring; then glucoamylase is added, then this obtained mixture is heated up to the temperature from 30 up to 70°C; grain particles are subject to grinding in order to obtain the medium size of max. 500 micrometer; then the mixture pH is brought to the level approximately from 7 to 10.5 and then alkaline protease is added for the purpose of protein solubisation. The obtained mixture goes through the sieve with the holes' diameter from 5 to 500 micrometer; then ultrafiltration process takes place with the use of membranes with their pore size from 20 kDa to 40 kDa and then nanofiltration takes place. This method's flaw is a necessity for using sophisticated and expensive equipment, long technological cycle for obtaining the high protein product (60-105 minutes, including 30-60 minutes for the grinding process and 30-45 minutes for hydrolysis) and the use of hazardous substances (hydrochloric or carboxylic acids and alkalis) in this technological process. Besides that this process requires great amounts of water use (from 8:1 to 11:1) and as a result a lot of centrate is formed. Such centrate is a waste product and additional equipment is required for its disposal.

Another technological solution (EP 0694609A2, incorporated herein by reference) implies obtaining a protein product from grains in the course of making beer. This product contains from 40 to 60% of proteins, from 12 to 18% of lipids, from 2 to 6% of fibre materials and from 1 to 4% of ash in dry mass. This method implies roller-grinding mill compression of the grains with simultaneous wet peeling of grain particles and further separation of the obtained product from the husk. The method has a flaw: valuable components are removed from brewer's grains in the course of compression with roller grinding mills. Besides that brewer's grains are not subject to grinding before compression because part of protein stays inside compressed husk particles therefore this protein is lost with the further husk removal. In order to enhance the husk removal efficiency the obtained mixture (liquid protein suspension) is rinsed with large quantities of water, then the obtained suspension is screened by means of screen plates. This rinsing and sieving process is repeated up to 5 times. As a result great amount of centrate is produced. Such centrate is a waste product and additional purification equipment is required for its disposal.

Thus, existing methods of processing brewer's grains aimed at obtaining protein powders or concentrates are characterized by complexity and duration of the protein production process, high output of centrate that is a waste product requiring additional equipment for its disposal.

Current methods are mainly aimed at obtaining a product in the form of powder or concentrate from brewer's grains without deriving in the course of technological processes a ready-for-use product that extends the range of dietary food products.

Therefore there is a necessity for creating methods of processing brewer's grains and obtaining the final product in the form of protein suspension rich in protein that can be widely used as a food and animal feed supplement.

### BRIEF DESCRIPTION OF DRAWINGS

All features of embodiments which are described in this disclosure are not mutually exclusive and can be combined with one another. For example, elements of one embodiment can be utilized in the other embodiments without further mention. A detailed description of specific embodiments is provided herein below with reference to the accompanying drawings in which:

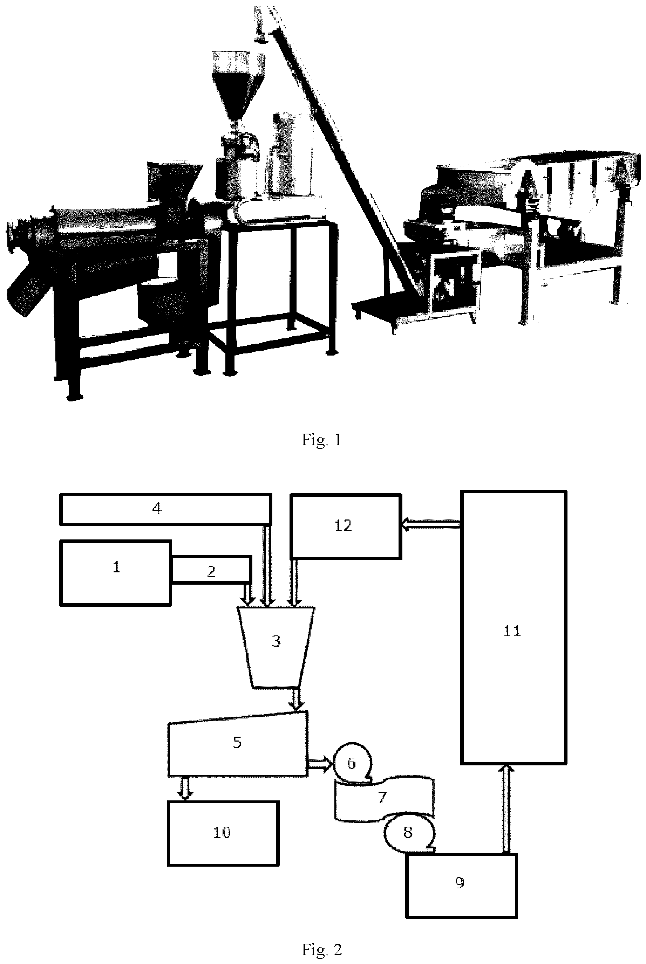

**Fig. 1** is a schematic representation of a production line showing a part of the pilot production line comprising a vibrating screen, a conveyor, a colloid mill and a screw extractor for obtaining protein suspension from brewer's grains.
**Fig. 2** is schematic representation of a production line for implementing a method according to one embodiment of the present technology, wherein: 1 indicates a vibrating screen, 2 indicates a conveyor, 3 indicates a colloid mill, 4 indicates a water pipeline, 5 indicates a screw extractor, 6 and 8 indicate impeller pumps, 7 indicates a vibration filter, 9 indicates a container (collection tank) for collecting protein suspension, 10 indicates a container (collection tank) for collecting husk, 11 indicates a block for processing the protein suspension in order to produce protein concentrate or protein isolate, 12 indicates a tank for centrate.

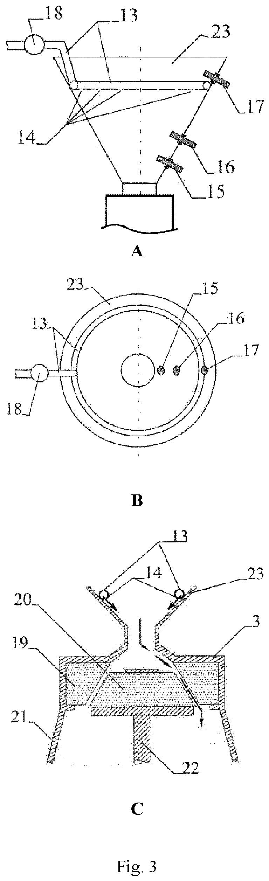

**Fig. 3** is a schematic representation of a colloid mill's loading tank. A indicates a layout of constructive elements in the colloid mill's loading tank, B indicates an overhead view of the loading tank, C indicates a cross-section diagram of the colloid mill, where 13 indicates a circular water pipeline, 14 indicates openings in the pipeline for water or centrate supply, 15, 16 and 17 indicate level-sensing devices, 18 indicates a control valve for water supply, 19 indicates a stator, 20 indicates a rotor, 21 indicates a stator shell, 22 indicates a rotor shaft and 23 indicates a loading tank of the colloid mill 3.

### DISCLOSURE OF TECHNOLOGY

The details of the present technology are explained below. This description is not intended to be a detailed catalog of all the different ways in which the technology may be implemented or all the features that may be added to the technology. For example, features illustrated with respect to one embodiment may be incorporated into other embodiments, and features illustrated with respect to a particular embodiment may be deleted from that embodiment. In addition, numerous variations and additions to the various embodiments suggested herein will be apparent. Hence, the following description is intended to illustrate some particular embodiments of the technology, and not to exhaustively specify all permutations, combinations and variations.

As used herein, the singular form "a," "an" and "the" include plural referents unless the context clearly dictates otherwise.

The recitation herein of numerical ranges by endpoints is intended to include all numbers subsumed within that range (e.g., a recitation of 1 to 5 includes 1, 1.25, 1.5, 1.75, 2, 2.45, 2.75, 3, 3.80, 4, 4.32, and 5).

The term "about" is used herein explicitly or not. Every quantity given herein is meant to refer to the actual given value, and it is also meant to refer to the approximation of such given value that would reasonably be inferred based on the ordinary skill in the art, including equivalents and approximations due to the experimental and/or measurement conditions for such given values. For example, the term "about" in the context of a given value or range refers to a value or range that is within 20%, preferably within 15%, more preferably within 10%, more preferably within 9%, more preferably within 8%, more preferably within 7%, more preferably within 6%, and more preferably within 5% of the given value or range.

The expression "and/or" is to be taken as specific disclosure of each of the two specified features or components with or without the other. For example, "A and/or B" is to be taken as specific disclosure of each of (i) A, (ii) B and (iii) A and B, just as if each is set out individually herein. The term "or" as used herein should be construed non-exclusively. For example, an embodiment of "a composition comprising A or B" would typically present an aspect with a composition comprising both A and B. "Or" should, however, be construed to exclude those aspects presented that cannot be combined without contradiction (e.g., a composition pH that is between 9 and 10 or between 7 and 8).

As used herein, the term "comprise" is used in its non-limiting sense to mean that items following the word are included, but items not specifically mentioned are not excluded.

As used herein, the expression "% wt" is calculated with respect to total dry solid weight.

Protein derived from brewer's grains is mostly used in bakery, pastry, sausage manufacture, sport and dietary nutrition. Protein derived from brewer's grains can also be used as a feed supplement in livestock farming, as a soil fertilizer in agriculture and others.

In some embodiments, the present technology relates to a product obtained in the course of processing brewer's grains in the form of suspension with the protein content of at least 50% wt (dry solid) with the nutritional energy value of about 250 ± 15 kcal, or less than about 250 ± 15 kcal, or more than about 250 ± 15 kcal with a simplified production method.

In some embodiments, the present technology relates to a suspension that is a ready-for-use product or an intermediate product from which concentrate, isolate or powder with the protein content of between about 90% wt and about 95% wt, or of less than about 90%, or of more than 95% wt can be derived in the course of further treatment.

In some occasions, the amount of centrate (fugate) being a waste product is minimal because of its use in the technological cycle for humidifying the raw material which is subject to processing. In some embodiments, the protein suspension obtained from brewer's grains has a humidity level of between about 90% and about 95% producing particle sizes of less than or equal to 0.5 mm.

In some embodiments, the present technology relates to a suspension composition comprising one or more of: i) a protein content of at least 50% dry wt; ii) a fat content that equals 5% or less than 5% dry wt; iii) a fibre content that equals 5% or less than 5% dry wt; iv) ash content of less than 1.5% dry wt; and v) amino acid content of at least 47% dry wt.

In some embodiments, the present technology relates to a method of obtaining protein suspension having the properties as defined herein. The method comprises loosing up the brewer's grains in order to obtain a homogeneous mass; removing impurities from the homogenous mass; humidifying the homogenous mass with subsequent while grinding and simultaneous homogenization in order to obtain a pasty mass (pulp) until the humidity level of less than or equal to 95%. In some instances, humidification is achieved by supplying water or centrate in the course of loading brewer's grains into the colloid mill. In some instances, the step of grinding is achieved by means of a colloid mill. After the ground husk is removed from the pulp food suspension is obtained with a protein content of at least 50% dry wt.

In some implementations, the method further comprises before producing the homogeneous mass, removing impurities with a vibrating screen with the hole size of between 6 mm and 10 mm and a screen vibration frequency ranging between 10 and 50 Hz and an amplitude of between 2 mm and 20 mm.

In some further embodiments, the raw materials are subjected to grinding with a colloid mill with the rotor frequency of between about 1,800 and about 3,200 rot./second in order to produce particles having an average size of between about 0.10 and about 0.9 mm. In the course of loading brewer's grains into the colloid mill water or centrate is supplied in order to provide homogeneous humidifying of the raw materials in volume. After grinding in the colloid mill, the ground husk is removed with a screw extractor for example. Then the food suspension is subject to additional vibrating filtration with the use of sieves (screens) with holes size of between about 0.1 mm and about 0.5 mm for the purpose of removing remaining husk particles.

In some implementations of these embodiments, the methods of the present technology may be carried out using apparatus comprising the following devices: a device for loosening and removing extra mechanical impurities, a grinding machine designed for humidifying raw materials in volume, grinding these materials in order to obtain a fraction of between about 0.1 mm and about 0.9 mm and homogenization; an extractor designed to do extra grinding of the mass in order to produce particles having 0.005-0.5 mm particles and its division into suspension and husk, a vibration filter with the hole size of between about 0.2 mm and about 0.5 mm designed to do additional separation of remaining husk particles from the suspension, a container for collecting the protein suspension. At the same time, a vibrating screen with a magnetic catcher with the hole size of between about 6 mm and about 10 mm and the screen vibration frequency from between about 10 and about 50 Hz and the amplitude of between about 2 and about 20 mm is used as a device for loosening and removing foreign impurities. A colloid mill equipped with a tool for water supply for the raw material humidifying is used as a grinding machine. Such colloid mill has a V-shaped (funnel-shaped) tank; (for the purpose of simultaneous humidifying of the raw materials) it contains a tool designed in the shape of a water pipeline with openings and nozzles located around the circumference of the tank in its upper part above the mark indicating the maximum tank load with raw materials. A screw extractor with the screw rotation rate from between about 2 rot./minute and about 8 rot./minute is used as an extractor. In addition, the apparatus has a block for concentrating the protein suspension equipped with an out channel for centrate connected with the grinding machine for humidifying the supplied raw materials.

In some embodiments, the produced protein suspension has a high protein content which is achieved by grinding brewer's grains with added water or centrate in the colloid mill, thorough separation of brewer's grain husk from the edible part in the screw separator with additional grinding of the processed mixture in the course of rubbing the mixture against the separator's filtering mesh with a screw. Besides that, centrate is not accumulated in the course of processing brewer's grains in order to obtain the final product in the form of suspension. In the process of producing concentrate, isolate or powder during the suspension processing, the produced centrate is recycled for humidifying the source raw materials and its quantity sent for disposal amounts to max. 1% from the manufacturing capacity of the brewer's grains processing line of about kg/1 min.

In some further embodiments, the brewer's grains with the humidity level of between about 70% and about 90% are subject to treatment in the course of 3 hours after they are produced (from the moment they are obtained as a waste product in the brewing industry). The temperature of such brewer's grains at the moment of delivery from the production facility varies from between about 2°C and about 80°C. The brewer's grains are loaded manually or by any mechanical method on a vibrating screen 1 (fig. 1, 2) with the hole size of between about 6 mm and about 10 mm equipped with a magnetic catcher where the brewer's grains are subject to removal of any mechanical and metallic extra particles in them. This process implies sieving of the brewer's grains with a vibration frequency ranging from between about 10 Hz and about 50 Hz and an amplitude of between about 2 mm and about 20 mm within between about 2 seconds and about 10 seconds in order to produce raw materials without lumps having homogeneous composition for the further processing stage when the material is subject to grinding. It is possible to obtain a homogeneous mass with removal of mechanical impurities in the course of loosening the brewer's grains not only by means of a vibrating screen but also by means of any device or a set of devices performing the mentioned functions. For the purpose of grinding, the conveyor 2 takes the loosened brewer's grains to the colloid mill 3 (fig. 3) or another grinding machine capable of producing particles having an average size of between about 0.1 mm and about 0.9 mm particles. At the same time the raw materials are loaded in the colloid mill's tank and water is added in order to provide homogeneous humidifying of the raw materials in volume that can be performed both in a continuous mode and in a pulsating mode. In some instances, the ratio of the supplied water varies from between about 0.5:1 an about 1:1. The amount and rate of water supply can be calculated in advance based on the measured initial humidity parameters of the brewer's grains delivered for processing taking into account humidity losses in the course of sieving the brewer's grains by means of the vibrating screen.

The humidity level of the brewer's grains in the colloid mill is within the range of between about 90% and about 95%. The colloid mill 3 performs its homogeneous mixing (and/or homogenization) in order to produce a homogeneous pasty mass (pulp) with the viscosity of between about 750 and about 1,400 cPa·s that moves to the screw extractor 5 where the mass is subject to additional grinding and division into suspension with a humidity level of between about 90 and about 95% and a viscosity of between about 1.5 and about 3 cPa·s and husk with the particle size ranging from between about 0.01 mm and about 1.0 mm and the humidity level of between about 60 and about 75%. The temperature of the brewer's grains in the colloid mill and the screw extractor can vary from between about 2°C and about 90°C. The brewer's grains can be supplied to the colloid mill with any known device, for example with a screw conveyor, or a belt conveyor, or a drag conveyor. Grinding of the brewer's grains in the colloid mill 3 takes place in the shell 21 between working surfaces of the rotor 20 and the stator 19, for example, in the course of rotation of the mill's rotor 20 at the rate of 1,800-3,200 rot./second what enables to produce thick, homogeneous, but sloppy consistency of the pulp for the maximum extraction of a nutritional fraction from the raw material at the screw extraction stage. It is preferable to use centrate produced in the course of the suspension's further processing (in case of its concentrating) in the colloid mill 3 instead of water as this may provide better extraction of the nutritional fractions remained in the centrate and may prevent disposal of the centrate before it is discharged to the sewerage system.

Water or centrate from blocks 5 or 12 is supplied to the V-shaped tank (receiving bunker) 23 of the colloid mill 3 via openings 14 of the water pipeline 13 located around the circumference of the tank in its upper part above the mark indicating the maximum tank load with raw materials. Amount of the supplied water or centrate can be regulated with the valve 18. In some instances, the openings 14 in the pipeline are uniformly spaced along its length in order to ensure homogeneous humidifying (thinning) of spent barley grains during the course of processing.

After the colloid mill 3, the pulp is processed in the screw extractor 5 with the screw rotation rate from 2 rot./minute to 8 rot./minute to quickly (e.g., within 1-2 seconds) separate the suspension from its waste product (i.e., barley's husk). The pasty mass (pulp) produced by the colloid mill 3 goes to the screw extractor 5 where it is separated from the husk in order to produce the main product - the suspension having a humidity level of less than about 95%, and a waste product, barley's husk, with a humidity level of between about 60% and about 75% and an average husk particle size of between about 1.0 mm and about 5.0 mm. After the treatment in the screw extractor 5, the suspension may contain between about 2% and about 5% of small husk particles having an average particle size ranging from between about 0.01 and about 1.0 mm. This suspension (with the impeller or another pump 6 designed for work with suspension with the impurity level of up to 5% represented by small fractions of max. 1.0 mm) goes to the next purification stage - vibration filter 7 with the filter holes of between about 0.2 mm and about 0.5 mm. That process practically guarantees complete removal of remaining husk in the suspension after the screw extraction stage. After the vibration filter 7, the suspension is pumped to the collection tank 9 with the impeller pump 8. The resulting protein suspension has a protein content of between about 50% and about 65% wt, which can be used as a food or animal feed supplement as well as being frozen for further use.

In some embodiments, the resulting protein suspension can be sent for further processing treatment to the block 11 for purpose of producing protein concentrate with a protein content of between about 60 and about 80% wt or a protein isolate with a protein content of at least about 80% wt.

Husk is a waste product of the brewer's grains and in the course of the screw extractor's operation husk falls in the collection bunker from where it is delivered to the collection tank 10 with the screw conveyor, or a spiral conveyor or any other conveyor. The machine can be used for producing protein suspension with the protein content of max. 50% wt, for example 40, 42, 47 and 49% wt (with a lower energy value) in case of respective settings of devices enabling to produce suspension particles of the upper level of the claimed size range (more than 0.5 mm). Such products can be used where there are no requirements for achieving the greatest possible results in protein content i.e. when it is used as animal feed.

### EXAMPLES

The examples below are presented to illustrate the practice of various embodiments of the present disclosure. They are not intended to limit or define the entire scope of this disclosure. The disclosure is not limited to the particular embodiments described and illustrated herein but includes all modifications and variations falling within the scope of the disclosure as defined in the appended embodiments.

### Example 1: Preparing Food Suspension

Food protein suspension in the amount of 337 litres may be produced by means of the method as described herein. For this purpose 260 kg of brewer's grains with an humidity level of 75.59% (original composition, energy value of 150 kcal) were manually loaded on the vibrating screen 1 represented by the vibrating table unit XFZ1020 with a single-level screen and 10 mm holes, with the table unit length of 2,000 mm, with the table unit width of 1,000 mm, with the vibration frequency of 20 Hz and the vibration amplitude of 8 mm.

From the vibrating screen 1 the mass by means of the belt conveyor 2 was supplied to the colloid mill 3 represented by the unit KDDJ-1,5 with the power capacity of 11 kW, with the rotation rate of 2,200 rot./minute of the rotor 20 that can also be equipped with a device for supplying drinking water from the block 4.

In the colloid mill, the brewer's grains were humidified by means of water with the design amount of 170 litres (0.67:1) that was supplied to the colloid mill at the rate of 15 litres per minute. At the same time, the humidified brewer's grains were grinded in order to obtain a faction of 0.1-0.9 mm. The process of supplying the source raw materials and water to the loading tank 23 of the colloid mill 3 was controlled by means of three level-sensing devices 15, 16 and 17 built in the shell of the loading tank 23 and a microcontroller located close to the level-sensing devices at the frame of the table on which the colloid mill was installed. At the same time one of the level-sensing device, the upper one, 17 was used for controlling the maximum possible load of the raw material in the bunker (85-90% in volume of the maximum capacity of the bunker); when this level was reached a command to stop the loading conveyor was given; the second level-sensing device, the middle one, 16 was used for controlling the minimum level of the loaded raw material (25-30% in volume of the maximum capacity of the bunker); when this level was reached a command to start the loading conveyor and to supply the raw material was given what provided for continuous operation of the colloid mill. The third level-sensing device, the lower one, 15 was installed near the loading bunker's bottom at the distance of 15 cm from the bottom and it was used for controlling the minimum possible load of the raw material in the bunker (10-15% in volume of the maximum capacity of the bunker); if this level was not reached the colloid mill stopped until another portion of the raw material was supplied.

After the colloid mill, the produced pulp with the viscosity of 900-1,200 cPa and the humidity level of 95% was delivered to the screw extractor 5 represented by the machine of KDLZ-1,5 model with the power capacity of 4 kW, with the rotation rate of 4.5-10 rot./minute.

The output was the main product, food suspension with the humidity level of 95% and the viscosity of 2.013 cPa, and a waste product, barley's husk, with the humidity level of 70.84%. By means of the impeller pump 6 with the power capacity of 0.25 kW with the rotation rate of 1,200 rot./minute the food suspension was delivered to the vibration filter 7 of XZS-1200-1S model with the power capacity of 0.75 kW with 0.3 mm openings.

After filtration by means of the impeller pump 8 with the power capacity of 0.25 kW with the rotation rate of 1,200 rot./minute the food suspension was pumped to the collection tank (container) 9. The husk naturally fell in the collection tank (container) 10. Thus, the food suspension with the humidity level of 93%, with the viscosity of 1.907 cPa and the particle size of 0.005-0.3 mm was produced. In order to assess its composition, 12 litres of suspension were dried in the spray-type drier HT-RY1500 during 8 hours at the temperature of 200°C until the humidity reached the level of 10% (capacity of this spray-type drier HT-RY1500 amounts to 1,500 ml of suspension per hour). The analysis showed that the obtained food suspension (sample 1) is characterized by nutritional energy value of 250 kcal and the following composition, % wt (dry solid) (Table 1):

**Table 1: Composition of food suspension obtained**

| Composition | Brewer's grains (original composition), % wt | Food suspension, % wt (sample 1) |
|---|---|---|
| Protein | 18.98 | 51.16 |
| Fats | 7.9 | 4.9 |
| Fibre | 13.6 | 4.5 |
| Ash | 2.2 | 0.8 |
| at the same time protein from brewer's gains is rich in the following amino acids: | | |
| Arginine | 1.07 | 4.27 |
| Lysine | 0.86 | 2.37 |
| Tyrosine | 0.61 | 2.55 |
| Phenylalanine | 1.23 | 3.57 |
| Histidine | 0.66 | 1.8 |
| Isoleucine | 0.79 | 3.79 |
| Leucine | 0.57 | |
| Methionine | 0.5 | 1.5 |
| Valine | 1.06 | 2.62 |
| Proline | 2.05 | 4.21 |
| Threonine | 0.77 | 2.26 |
| Serine | 0.89 | 1.79 |
| Alanine | 0.94 | 3.6 |
| Glycine | 0.79 | 2.19 |
| Cystine | 0.46 | 1.91 |
| Glutamic acid | 4.57 | 8.63 |
| Asparaginic acid | 1.35 | 2.06 |
| Total amount of amino acids | 19.17 | 49.12 |

The total time for processing 260 kg of brewer's grains amounted to 25 minutes. Thus, the protein suspension produced had a high protein content with preservation of the amino acid composition of the brewer's grains and a low content of fats and fibres. The time from loading raw material in the machine to obtaining the final product in the form of suspension, for example when calculated for 100 kg of brewer's grains, took between about 5 and 10 minutes with the equipment's capacity from 20 to 500 tonnes/day; at the same time, the amount of centrate being a waste product and subject to disposal was minimal and it was equal to max. 1% of the capacity of the brewer's grain processing line of kg/1 minute.

Brewer's grains delivered from five different manufacturing facilities (sample) were processed by this equipment in accordance with the method of the present technology. The quantitative content of ingredients in the brewer's grains compositions was different from the original composition specified in table 1 within the limit of 1-5%. Table 2 shows compositions of protein suspensions with the optimal content of key components.

**Table 2: Compositions of protein suspensions**

| Parameters | Food suspension | | | | |
|---|---|---|---|---|---|
| | sample 2 | sample 3 | sample 4 | sample 5 | sample 6 |
| nutritional energy value (dry solid) | 245 kcal | 260 kcal | 258 kcal | 255 kcal | 265 kcal |
| humidity | 91 % | 93 % | 92% | 93 % | 95% |
| particle size | 0.005-0.5 mm | 0.005-0.1 mm | 0.005-0.3 | 0.005-0.4 mm | 0.005-0.1 mm |

| Composition | content (% wt) | | | | |
|---|---|---|---|---|---|
| Protein | 51.1 | 62.19 | 58.3 | 55.4 | 64.7 |
| Fats | 3.7 | 4.9 | 3.2 | 3.8 | 4.7 |
| Fibre | 2.4 | 3.8 | 4.2 | 3.1 | 4.5 |

| Parameters | Food suspension | | | | |
|---|---|---|---|---|---|
| | sample 2 | sample 3 | sample 4 | sample 5 | sample 6 |
| Ash | 0.4 | 0.82 | 0.56 | 0.7 | 1.0 |

| Amino acid composition: | | | | | |
|---|---|---|---|---|---|
| Arginine | 3.93 | 4.27 | 4.6 | 4.0 | 5.3 |
| Lysine | 1.95 | 3 | 2.72 | 2.87 | 3.17 |
| Tyrosine | 2.15 | 3.85 | 2.53 | 2.23 | 3.72 |
| Phenylalanine | 3.5 | 4.97 | 4.47 | 3.68 | 4.17 |
| Histidine | 2.1 | 2.9 | 2.1 | 1.85 | 2.1 |
| Isoleucine / Leucine | 2.23 | 3.79 | 2.05 | 2.89 | 3.82 |
| Methionine | 2.43 | 2.55 | 2.1 | 1.97 | 2.1 |
| Valine | 2.84 | 2.62 | 2.9 | 2.75 | 3.16 |
| Proline | 3.85 | 4.73 | 4.1 | 3.95 | 5.1 |
| Threonine | 1.79 | 3.12 | 3.7 | 3.17 | 3.7 |
| Serine | 1.98 | 2.3 | 2.4 | 1.95 | 2.4 |
| Alanine | 2.84 | 4.1 | 4.3 | 3.97 | 4.3 |
| Glycine | 2.98 | 2.49 | 3.1 | 2.94 | 3.1 |
| Cystine | 2.62 | 2.1 | 2.4 | 1.95 | 2.4 |
| Glutamic acid | 7.7 | 9.8 | 8.5 | 7.94 | 10.3 |
| Asparaginic acid | 2.35 | 3.2 | 2.6 | 2.27 | 3.4 |
| Total amount of amino acids | 47.24 | 59.79 | 54.57 | 50.38 | 62.24 |

**Table 3: Parameters of processing brewer's grains (samples 2-6).**

| Equipment | Processing parameters | | | | |
|---|---|---|---|---|---|
| | sample 2 | sample 3 | sample 4 | sample 5 | sample 6 |
| Colloid mill/rotor rotation rate (rot./second) | 1,800 | 3,000 | 2,500 | 2,000 | 3,200 |
| Vibrating screen screen hole size (mm) / vibration frequency (Hz and mm) | 10/10, 15 | 5/40, 10 | 7/30, 20 | 8/20, 15 | 6/50, 8 |
| Screw extractor / rotation rate (rot./second) | 3 | 8 | 5 | 7 | 10 |
| Filtration / hole size (mm) | 0.5 | 0.1 | 0.3 | 0.4 | 0.1 |

Based on the above-given data it can be concluded that a protein product in the form of suspension with high protein content is produced despite the fact that various plants use different types of barley, have various technologies for manufacturing brewer's malt, various malt mix recipes for manufacturing beer etc. The two-stage processing of brewer's grains (in the colloid mill and the screw extractor) without using multi-step compression processes and thermochemical treatment enables to obtain a protein product with the humidity level of max. 95% and the particle size of max. 0.5 mm with the protein content of min. 50.0% wt (dry solid) and without gluten.

This method of producing protein suspension is universal and enables to preserve all valuable biologically active agents of the source brewer's grains. The rich chemical composition of brewer's grains with the minimal content of hydrocarbons predetermines its prospects in the food-processing industry, in particular its use as an albuminous vitamin-mineral additive in manufacturing of different products.

## Claims

1. Protein suspension produced from brewer's grains is **characterized in that** it has the humidity level of max. 95%, the particle size of max. 0.5 mm and it contains proteins, fats, fibre and ash; at the same time, the protein content in such suspension amounts to min. 50% wt (dry solid).

2. Protein suspension as per clause 1 is **characterized in that** the quantitative content of ingredients (dry solid) amounts to the following % wt: fats - max. 5.0; fibre - max. 5.0; ash - max. 1.5; at the same time, the amino acids content amounts to min. 37.0.

3. The method for producing protein suspension as per clause 1 is **characterized in that** the source brewer's grains are loosened in order to obtain a homogeneous mass, mechanical impurities are removed, then the mass is subject to grinding by means of a colloid mill with addition of water or centrate in order to produce a pasty mass with the humidity level of max. 95%, then the ground husk is removed from the mass and the final product represented by food suspension with the protein content of min. 50% wt (dry solid) is produced.

4. The method as per clause 1 is **characterized in that** the raw material is subject to grinding by means of a colloid mill in order to produce 0.1-0.9 mm particles.

5. The method as per clause 1 is **characterized in that** the mass grinding and homogenization in the colloid mill is performed in the course of rotation of the mill's rotor at the rate of 1,800-3,200 rot./second.

6. The method as per clause 1 is **characterized in that** the ground husk in the colloid mill is removed by means of a screw extractor.

7. The method as per clause 1 is **characterized in that** in the course of loading brewer's grains into the colloid mill water or centrate is supplied in order to provide for homogeneous humidifying of the raw materials in volume.

8. The method as per clause 1 is **characterized in that** before producing a homogeneous mass, foreign impurities are removed by means of a vibrating screen with the hole size of 6-10 mm, and the screen vibration frequency from 10 to 50 Hz and the amplitude of 2-20 mm.

9. The method as per clause 1 is **characterized in that** the food suspension is subject to additional vibrating filtration with the use of screens with the hole size of 0.1-0.5 mm for the purpose of removing remaining husk particles.

10. The machine for producing protein suspension as per clause 1 is **characterized in that** it is equipped with the following components connected in a particular sequence: a device for loosening brewer's grains and removing foreign impurities from them; a grinding machine designed with a possibility of humidifying raw materials in volume, grinding these materials and producing a pasty mass; an extractor designed with a possibility of grinding the mass in order to produce 0.01-0.5 mm particles and its division into suspension and husk; a vibration filter with the hole size of 0.1-0.5 mm designed with a possibility of additional separation of remaining husk particles from the suspension; a container for collecting the protein suspension.

11. The unit as per clause 10 is **characterized in that** it is equipped with a vibrating screen with a magnetic catcher for loosening and removing mechanical impurities.

12. The unit as per clause 11 is **characterized in that** it is equipped with a vibrating screen with the hole size of 6-10 mm and the screen vibration frequency from 10 to 50 Hz and the amplitude of 2-20 mm.

13. The unit as per clause 10 is **characterized in that** it is equipped with a grinder represented by a colloid mill with a device for liquids supply for humidifying raw materials designed for grinding the source brewer's grains in order to produce 0.1-0.9 mm fraction.

14. The unit as per clause 13 is **characterized in that** the colloid mill is equipped with a V-shaped tank and for the purpose of uniform humidifying of the raw materials it contains a tool designed in the form of a water pipeline with openings and nozzles located around the circumference of the tank in its upper part above the mark indicating the maximum tank load with raw materials.

15. The unit as per clause 10 is **characterized in that** it is equipped with a screw extractor with the screw rotation rate from 2 rot./minute to 8 rot./minute used as an extractor.

16. The unit as per clause 10 is **characterized in that** it is equipped with an additional block for concentrating the protein suspension equipped with an out channel for centrate connected with the grinding machine for humidifying the supplied raw materials.
